(19) Europäisches Patentamt
European Patent Office
Office européen des brevets

(11) **EP 3 405 304 B1**

## (12) FASCICULE DE BREVET EUROPEEN

(45) Date de publication et mention
de la délivrance du brevet:
**02.10.2019 Bulletin 2019/40**

(21) Numéro de dépôt: **17706280.9**

(22) Date de dépôt: **23.01.2017**

(51) Int Cl.:
*A61K 33/24* (2019.01)        *B22F 1/00* (2006.01)
*B22F 9/24* (2006.01)

(86) Numéro de dépôt international:
**PCT/FR2017/050131**

(87) Numéro de publication internationale:
**WO 2017/125695 (27.07.2017 Gazette 2017/30)**

(54) **NANOPARTICULES D'OR ET PROCÉDÉ ÉCOLOGIQUE DE PRÉPARATION**

GOLDNANOPARTIKEL UND ÖKOLOGISCHES VERFAHREN ZUR HERSTELLUNG

GOLD NANOPARTICLES AND ECOLOGICAL METHOD OF PRODUCTION

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **22.01.2016 FR 1650520**

(43) Date de publication de la demande:
**28.11.2018 Bulletin 2018/48**

(73) Titulaires:
• **Torskal**
**97490 Sainte-Clotide (FR)**
• **Morel, Anne-laure**
**97438 Sainte-Marie (FR)**

(72) Inventeur: **MOREL, Anne-Laure**
**97438 Sainte-Marie (FR)**

(74) Mandataire: **IP Trust**
**2, rue de Clichy**
**75009 Paris (FR)**

(56) Documents cités:
• **VINEET KUMAR ET AL: "Plant-mediated
synthesis of silver and gold nanoparticles and
their applications", JOURNAL OF CHEMICAL
TECHNOLOGY AND BIOTECHNOLOGY, vol. 84,
no. 2, 1 février 2009 (2009-02-01), pages 151-157,
XP055306383, ISSN: 0268-2575, DOI:
10.1002/jctb.2023**
• **DIPESH KR. DAS ET AL: "Biosynthesis of
stabilised gold nanoparticle using an aglycone
flavonoid, quercetin", JOURNAL OF
EXPERIMENTAL NANOSCIENCE, vol. 8, no. 4, 1
mai 2013 (2013-05-01), pages 649-655,
XP055306388, GB ISSN: 1745-8080, DOI:
10.1080/17458080.2011.591001**

## Description

Domaine de l'invention

La présente invention se rapporte à des nanoparticules d'or et à leurs applications médicales et cosmétiques. La présente invention se rapporte également à un procédé écologique de préparation desdites nanoparticules à partir d'extraits de plantes et d'une solution aqueuse de sels d'or.

Les nanoparticules susceptibles d'être obtenues par le procédé de l'invention peuvent être utilisées dans plusieurs applications, notamment dans le médical ou la cosmétique, par exemple en tant que catalyseur, senseur, marqueur pour microscopie électronique, thérapie génique, vecteur de principe actif, diagnostic, radiothérapie, thérapie par hyperthermie, imagerie...

Etat de la technique

Les nanoparticules sont de plus en plus utilisées dans la médecine et la cosmétique. Celles-ci peuvent être de nature métallique ou non et ont une taille n'excédant pas quelques centaines de nanomètres. La nature des nanoparticules leur confère des propriétés propres.

Les nanoparticules d'or présentent un intérêt fort en nanomédecine. Celles-ci ont l'avantage de posséder une résonance des plasmons de surface unique qui les rend particulièrement intéressantes en médecine (diagnostique, thérapie, recherche...). De plus, elles ne s'oxydent pas, sont inertes et biocompatibles. Les nanoparticules d'or peuvent être également couplées à des molécules biologiques telles que des anticorps, des peptides ou autres, voire des molécules inorganiques.

Classiquement, les nanoparticules métalliques étaient synthétisées par des procédés chimiques dont les réactifs ou produits de réaction pouvaient être toxiques. Généralement, l'agent réducteur utilisé était le citrate (Turkevich, 1951). Ces procédés nécessitaient des lavages des nanoparticules afin de tenter d'éliminer des agents toxiques. Ces nanoparticules, du fait de leur procédé de synthèse pouvaient entraîner des problèmes de stabilité, de réactivité, de biocompatibilité voire même les rendaient impropres à la conjugaison avec des biomolécules, les réactifs de synthèse entraînant une inactivation de la biomolécule.

Il a été nécessaire de développer de nouveaux procédés de synthèse, plus respectueux de l'environnement et améliorant la biocompatibilité, la stabilité et la réactivité des nanoparticules d'or.

Il est ainsi connu de l'état de la technique des procédés respectueux de l'environnement utilisant des extraits de plantes pour réduire les sels d'or.

La demande de brevet WO2005/095031 décrit un procédé pour la synthèse de nanoparticules mono et bimétalliques de tailles et de formes différentes, par réaction de sels métalliques avec des extraits réducteurs de différentes parties de plantes à une température comprise entre 15° C et 100 °C. Les plantes dans cette demande de brevet sont par exemple des variétés de cannelle, de citron, de jasmin, de camomille, de rose...

Le brevet US8333994 décrit des nanoparticules d'or biocompatibles stabilisées avec des extraits de plantes riches en polyphénols ou riches en flavonoïdes ou des composés réactifs phytochimiques. Le procédé est respectueux de l'environnement. Dans ce brevet, une solution aqueuse contenant des sels d'or sont mixés avec les extraits de plantes riches en polyphénols ou riches en flavonoïdes. La solution de sels d'or est mixée par exemple avec du thé noir, du curcuma ou de la cannelle.

Kumar et al. (2009, J Chem Technol Biotechnol) décrit un procédé écologique de préparation de nanoparticules d'or biocompatibles et stables comprenant la préparation d'au moins un extrait de plante riche en flavonoïdes puis le mélange d'au moins un desdits extraits de plante avec une solution aqueuse d'au moins un sel d'or. Les plantes dont proviennent les extraits riches en flavonoïdes sont par exemple le géranium, la citronnelle, la cannelle, l'aloé véra...

Inconvénients de l'art antérieur

Les procédés requérant des produits chimiques présentent l'inconvénient de ne pas être respectueux de l'environnement et les nanoparticules d'or obtenues peuvent poser des problèmes de stabilité, de réactivité, de toxicité ou de biocompatibilité en particulier lors de leurs injections ultérieures dans l'organisme du fait d'une dissociation des molécules (toxiques) les stabilisant en solution. Ces molécules peuvent alors être responsables des effets secondaires constatés.

Les procédés de l'état de la technique utilisant des extraits de plantes conduisent majoritairement à la génération de particules d'or sphériques instables en solution aqueuse. Il est connu que la forme des nanoparticules influence directement la capacité des nanoparticules à atteindre le microenvironnement des tumeurs et donc l'efficacité du traitement (Stylianopoulos et al). La taille et la forme des nanoparticules sont influencées par la composition en antioxydants des extraits de plantes. Trois facteurs déterminent la taille et forme des nanoparticules : le pouvoir réducteur des polyphénols, leur concentration dans l'extrait, et la nature ou la concentration des agents stabilisants (Elia et al, 2014). Très souvent, les procédés d'extraction écologiques conduisent à une accumulation d'agents polyphénoliques de natures différentes (acides phénoliques, catéchine, épicatéchine, anthocyane flavonoïdes, tanins, etc.) qui ne permettent pas la stabilisation à long terme des nanoparticules en solution (Krishnaswamy et al, 2014).

Afin d'augmenter l'efficacité de ces nanoparticules, tant d'un point de vue de leur capacité à atteindre les cellules-cibles que pour améliorer leur efficacité, il est primordial de trouver des procédés respectueux de, l'environnement permettant d'obtenir des nanoparticules d'or

monodispersées, stables, plus performantes pour générer de l'hypertehremie, biocompatibles et dont la forme est maîtrisée et uniforme.

Solution apportée par l'invention

La présente invention se propose de remédier aux inconvénients de l'art antérieur par un procédé écologique de préparation de nanoparticules d'or biocompatibles et stables consistant à réduire des sels métalliques à l'aide d'au moins un extrait d'une plante riche en flavonoïdes, en particulier un extrait de *Hubertia ambavilla* ou de *Hypericum lanceolatum*. Les nanoparticules d'or ainsi obtenues présentent des propriétés améliorées par rapport à celles des nanoparticules de l'art antérieur.

**DESCRIPTION DETAILLEE DE L'INVENTION**

Un premier objet de l'invention concerne un procédé écologique de préparation de nanoparticules d'or biocompatibles et stables comprenant :

   a. La préparation d'au moins un extrait de plante riche en flavonoïdes
   b. Le mélange d'au moins un desdits extraits de plante avec une solution aqueuse d'au moins un sel d'or

caractérisé en ce que l'extrait de plante riche en flavonoïdes est un extrait de *Hubertia ambavilla* ou de *Hypericum lanceolatum.*

*Hubertia ambavilla* est une espèce endémique de l'île de la Réunion et de l'île Maurice. Ce petit arbuste est particulièrement abondant à la Réunion où on le trouve principalement entre 1000 et 2000 m d'altitude. Cette plante est utilisée dans la médecine traditionnelle réunionnaise en tisane pour soigner les gastro-entérites chez le nourrisson ou en décoction pour soigner les problèmes cutanés (bourbouille, eczéma, brûlures, démangeaisons, plaies, inflammations...). Cette plante aurait aussi des propriétés contre l'ulcère de l'estomac et des propriétés antivirales contre l'herpès et le virus de la poliomyélite.

*Hypericum lanceolatum* est une espèce de millepertuis arborescent originaire de l'île de la Réunion. Elle est aussi connue sous le nom de fleur jaune. Elle est également retrouvée en Afrique, de la Guinée équatoriale à la Tanzanie. L'huile essentielle de fleurs de cette plante est utilisée en phytothérapie. Cette plante est utilisée dans la médecine traditionnelle réunionnaise sous forme de tisane pour soulager les brûlures d'estomac, contre les affections urinaires, pour soulager et régulariser les règles douloureuses ou encore contre la fièvre. Ses propriétés antiradicalaires et antioxydantes sont prouvées.

Selon des modes alternatifs de réalisation de l'invention, l'extrait de plante riche en flavonoïdes peut être un extrait total brut de ladite plante ou un totum de flavonoïdes de ladite plante, notamment :

- un extrait total brut de *Hubertia ambavilla.*
- un extrait total brut de *Hypericum lanceolatum.*
- un totum de flavonoïdes de *Hubertia ambavilla.*
- un totum de flavonoïdes de *Hypericum lanceolatum.*

Avantageusement, l'extrait de plante comprend des flavonoïdes choisis parmi la rutine, la quercétine, l'hyperoside et l'isoquercétine ou une combinaison d'au moins deux parmi ceux-ci.

Selon un mode de réalisation particulier, l'extrait de plantes comprend de la rutine et de la quercétine. Avantageusement, l'extrait de plante comprenant de la rutine et de la quercétine est un totum de flavonoïdes. Le totum flavonoïdes comprenant de la rutine et de la quercétine est un totum de flavonoïdes extrait de *Hypericum lanceolatum.*

Selon un autre mode de réalisation, l'extrait de plantes comprend de l'hyperoside et de l'isoquercétine. Avantageusement, l'extrait de plante comprenant de l'hyperoside et de l'isoquercétine est un totum de flavonoïdes. Le totum de flavonoïdes comprenant de l'hyperoside et de l'isoquercétine est un totum de flavonoïdes extrait de *Hubertia ambavilla.*

Avantageusement, le ratio molaire de flavonoïdes aux sels d'or est de 21 lorsque le procédé de préparation de nanoparticules d'or selon l'invention consiste à mélanger la solution de sels d'or avec un totum de flavonoïdes extrait de *Hubertia ambavilla* ou de *Hypericum lanceolatum.*

Dans un mode de réalisation préféré, l'extrait de plante riche en flavonoïdes mis en oeuvre dans le procédé de préparation de nanoparticules d'or est un extrait brut de *Hubertia ambavilla* ou de *Hypericum lanceolatum.*

Le procédé selon l'invention est rapide. Les extraits de plantes et totum de flavonoides jouent également le rôle de stabilisants des nanoparticules une fois formées. Il comporte donc un nombre réduit d'étapes. Une fois l'extrait de plante obtenu, le procédé se fait en une étape et en moins d'une minute. Ceci est beaucoup plus rapide que les procédés chimiques de l'art antérieur (Frens et al., 1973, Zhao, 2013). A titre d'exemple, la composition chimique des extraits bruts d'*Hubertia ambavilla* demeure très stable en dépit des variabilités, liées aux saisons, généralement constatées dans les autres espèces florales (Hyperoside/isoquercetine = 30). Les composés majoritaires sont des couples de molécules, de ratio stable, qui participent ainsi à la stabilisation des nanoparticules sans addition d'agent stabilisant.

Les nanoparticules susceptibles d'être obtenues par le procédé selon l'invention ont un diamètre compris entre 5 et 100 nm, avantageusement entre 10 et 50 nm.

Deux types de nanoparticules sont obtenus en fonction de la nature de l'extrait de plante mis en oeuvre dans le procédé de préparation.

Les nanoparticules obtenues par mélange d'un totum de flavonoïdes de plante avec une solution aqueuse d'au moins un sel d'or sont sphériques. Avantageusement, ces nanoparticules sphériques ont un diamètre de 15 nm.

Les nanoparticules obtenues par mélange d'un extrait total brut de plante avec une solution aqueuse d'au moins un sel d'or sont anisotropiques en forme de fleur. Avantageusement, ces nanoparticules en forme de fleur ont un diamètre de 40 à 80 nm.

Les deux types de nanoparticules obtenues selon le procédé de l'invention ont des propriétés améliorées par rapport aux nanoparticules décrites dans l'état de la technique.

Un deuxième objet de l'invention concerne des nanoparticules d'or anisotropiques en forme de fleur comprenant un mélange d'or et d'extrait brut de *Hubertia ambavilla* ou de *Hypericum lanceolatum.*

Cette forme particulière « de fleur » est induite par la polymérisation en solution des polyphénols qui sont responsables à la fois de la réduction des sels d'or, de la nucléation et croissance des nanoparticules dans l'espace selon les trois axes x, y, z (autrement dit en trois dimensions), et de leur stabilisation. Les inventeurs ont montré que la quantité de chaleur produite par ces nanoparticules en forme de fleur, lorsqu'elles sont irradiées par infrarouge, est supérieure à celles des nanoparticules sphériques décrites dans l'art antérieur, et également à celle des nanoparticules sphériques produites en appliquant le procédé objet de l'invention. Cette propriété résulte certainement des interactions électromagnétiques du faisceau incident avec les différents plasmons de surface de la nanoparticule en forme de fleur, permettant une augmentation de la chaleur restituée. Ainsi, ces nanoparticules en forme de fleur ont une efficacité augmentée pour une même dose d'irradiation. Leur réactivité accrue par rapport aux nanoparticules de l'art antérieur suggère une meilleure efficacité thérapeutique. Ces nanoparticules sont de surcroît stables et monodispersées. Leur biodistribution a également été étudiée par les inventeurs et les premiers résultats montrent qu'elles sont capables de s'accumuler dans différents organes, en particulier dans le foie, le poumon et le rein.

Les nanoparticules en forme de fleur de l'invention, obtenues par un procédé de chimie verte, présentent l'avantage de ne contenir aucune trace de particules toxiques, contrairement aux nanoparticules décrites dans l'art antérieur et obtenues par procédé chimique.

Comme mentionné précédemment, ces nanoparticules en forme de fleur sont obtenues par le procédé selon l'invention lorsque que l'extrait de plante est un extrait brut de *Hubertia ambavilla* ou de *Hypericum lanceolatum.*

Un troisième objet de l'invention concerne l'utilisation des nanoparticules susceptibles d'être obtenues selon le procédé de l'invention dans des applications cosmétiques, diagnostiques et thérapeutiques.

Comme présenté dans la partie expérimentale, les nanoparticules obtenues par le procédé de l'invention sont particulièrement réactives, médiatrices d'hyperthermie et présentent une biodistribution intéressante pour des applications diagnostiques et thérapeutiques.

En cosmétique, elles peuvent entrer dans la formulation de produits cosmétiques pour la peau, les cheveux et les ongles.

De plus, les nanoparticules susceptibles d'être obtenues par le procédé objet de l'invention sont solubles dans l'eau et sont des nanothéranostiques, c'est à dire qu'elles peuvent être utilisées en tant qu'agent diagnostique et/ou en tant qu'agent thérapeutique.

Les nanoparticules susceptibles d'être obtenues par le procédé de l'invention sont présentes dans certains tissus pouvant développer des cancers. Elles peuvent ainsi être utilisées à la fois dans des techniques d'imagerie médicale ou vétérinaire, et dans des techniques de traitement médical ou vétérinaire, par exemple l'hyperthermie par photothérapie.

Avantageusement, les cellules qui seront ciblées dans ces méthodes d'imagerie ou de diagnostique seront les cellules du foie, du poumon et du rein.

Du fait de leurs propriétés, les nanoparticules susceptibles d'être obtenues par le procédé objet de l'invention sont particulièrement adaptées au traitement du cancer.

De manière générale, les nanoparticules d'or sont biocompatibles et possèdent des propriétés optiques permettant l'utilisation dans le domaine médical (Jabeen F, *et al.,* 2014 ; Jiang Y., *et al.,* 2015 ; Shanmugam V., *et al,* 2014). La possibilité d'utiliser les nanoparticules en imagerie *in vivo* à l'aide des ultra-sons a déjà été montré (Arifin DR, et *al.,* 2011). Les nanoparticules sont également utilisées dans le cadre des thérapies du cancer par hyperthermie via les infra-rouges ; l'intérêt de cette approche réside dans son aspect peu invasif et dans la facilité de sa mise en oeuvre (Song J,. *et al.,* 2016).

Des essais ont été menés pour obtenir des nanoparticules nanothéranostiques, utilisant la photothermie et l'imagerie ; ceci notamment en combinant des coquilles d'or avec des polymères, ou encore avec de l'acide lactique (Ke H et al, 2014). Actuellement, les nanoparticules d'or de toutes formes et des toutes tailles sont fréquemment utilisées en thérapie via l'hyperthermie en raison de leur photosensibilité liée à la résonnance plasmonique (Huang, X et al , 2011). Cette propriété a été mise à profit dans le traitement du cancer. L'application d'un laser sur les nanoparticules localisées sur le site tumoral provoque une augmentation de la chaleur en local. Cela provoque la dénaturation des protéines et des membranes cellulaires dans l'environnement immédiat des nanoparticules, ce qui induit la mort des cellules cancéreuses (Shibu ES, et al, 2013).

Les avancées de l'utilisation des nanoparticules d'or dans le traitement par hyperthermie des tissus cancéreux sont liées aux capacités du métal à absorber le rayonnement infrarouge puis à émettre, en réponse, dans un second temps, des rayonnements infra-rouges. Pour mettre en oeuvre une telle approche thérapeutique, il convient d'étudier l'environnement de la tumeur à traiter pour définir les rayonnements adéquats et les températures nécessaires et admissibles. Récemment, des travaux ont été menés en mathématiques pour modéliser les effets photothermiques des nanoparticules dans les cellules et les tissus (Nabil, M *et al.,* 2015 ; Peeters, S *et al.,* 2012). Les différences constatées dans les gammes de températures en fonction des nanoparticules considérées ont mené à une modification de l'équation d'Arrhénius (Li, D *et al* , 2014 ; Clark, C.D. *et al.,* 2011). Cela permet de mieux sélectionner les tailles de nanoparticules et les longueurs d'ondes des rayonnements pour provoquer une hyperthermie appropriée.

Les nanoparticules selon l'invention apparaissent comme particulièrement adaptées au traitement du cancer. En effet, ces nanoparticules d'or sont issues d'un procédé de synthèse dit « de chimie verte » ne comportant pas de composés toxiques. De plus, les nanoparticules d'or obtenues selon le procédé objet de l'invention ne présentent pas de trace de produit toxique en leur sein. Ainsi, contrairement à d'autres nanoparticules, l'injection de ces nanoparticules ne s'accompagne pas d'une injection de produits toxiques dans l'organisme, même à l'état de traces. Elles sont de ce fait très sûres.

Contrairement aux agents nanothéranostiques actuels qui ne ciblent pas spécifiquement les cibles cancéreuses, les nanoparticules d'or obtenues selon le procédé objet de l'invention sont retrouvées préférentiellement dans les poumons, le foie et les reins, qui sont des tissus dans lesquels les cancers sont fréquents, notamment le foie. De plus, elles peuvent être fonctionnalisées à l'aide anticorps spécifiques de cibles antigéniques des tissus cancéreux d'intérêt comme cela a déjà été proposé (Xu L. *et al,* 2017). Elle peuvent être également fonctionnalisées par des molécules non spécifiques des tissus cibles mais favorisant l'adressage des nanoparticules sur lesdits tissus cibles ; ces molécules non spécifiques pouvant être notamment des molécules entrant dans le métabolisme particulier des cellules dudit tissu cible.

Enfin, les nanoparticules d'or obtenues selon le procédé objet de l'invention présentent un potentiel hyperthermique élevé qui est avantageux du fait que la dose d'irradiation à délivrer sera d'autant moins forte.

Du fait de toutes ces propriétés, les nanoparticules d'or obtenues selon le procédé objet de l'invention sont des candidats sérieux pour des applications antitumorales.

Ainsi, dans un mode de réalisation particulier, l'invention concerne l'utilisation des nanoparticules susceptibles d'être obtenues par le procédé objet de l'invention pour traiter le cancer. Avantageusement, les cancers traités par ces nanoparticules seront le cancer du foie, du poumon et du rein.

Dans un autre mode de réalisation, les nanoparticules objet de l'invention peuvent être fonctionnalisées par tout type de molécules permettant leur ciblage dans un organe, un tissu ou un type cellulaire particulier, en particulier à l'aide de protéines, en particulier d'anticorps, de polymères ou de sucres.

La présente invention concerne également une méthode de diagnostique et/ou de traitement comprenant les étapes de :

- Administration de nanoparticules susceptibles d'être obtenues par le procédé objet de l'invention
- Irradiation des cellules ou du tissu cible(s) avec une dose appropriée de radiations électromagnétiques, par exemple des infrarouges
- Détection et / ou destruction des cellules ou du tissu irradié(es) ayant incorporé(es) lesdites nanoparticules.

Avantageusement les cellules ou le tissu cible(s) sont des cellules ou un tissu cancéreux.

Avantageusement, les nanoparticules utilisées dans ces méthodes sont anisotropiques, en forme de fleur.

Les nanoparticules obtenues par le procédé sont monodispersées. Elles sont administrables chez l'Homme ou l'animal, par exemple *per os* ou par voie intraveineuse. Elles peuvent être taguées ou fonctionnalisées pour un meilleur adressage aux cellules ou tissus cibles. Elles peuvent être couplées à des techniques mettant en jeu des ondes électromagnétiques telles que CT-Scan, IRM, SERS RAMAN, pour détecter et/ou traiter et/ou suivre l'évolution de diverses pathologies dont différents types de cancer.

La présente invention sera mieux comprise à la lumière des exemples qui suivent.

## DESCRIPTION DES FIGURES

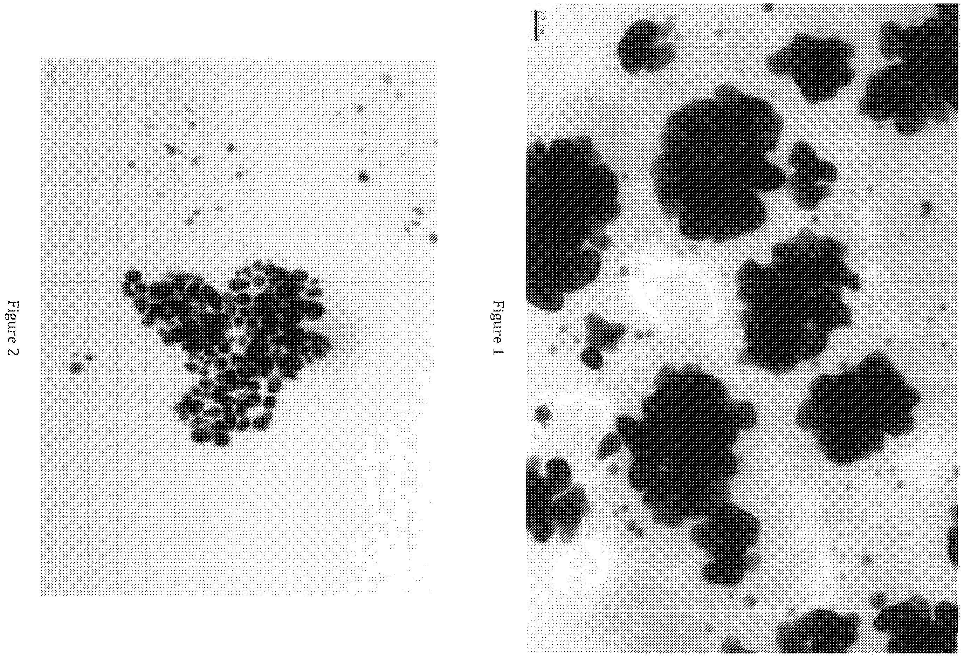

La figure 1 présente des clichés pris en microscopie électronique à transmission de nanoparticules d'or obtenues selon le procédé de l'invention à partir de sels d'or réduits par un extrait total brut de feuilles de *Hubertia ambavilla.* Les nanoparticules obtenues ont un diamètre de 40 nm et sont anisotropiques en forme de fleurs. Elles sont monodispersées.

La figure 2 présente des clichés pris en microscopie électronique à transmission de nanoparticules d'or obtenues selon le procédé de l'invention à partir de sels d'or réduits par un totum de flavonoïdes de fleurs de *Hubertia ambavilla.* Les nanoparticules obtenues ont un diamètre de 15 nm et sont sphériques. Elles sont monodispersées.

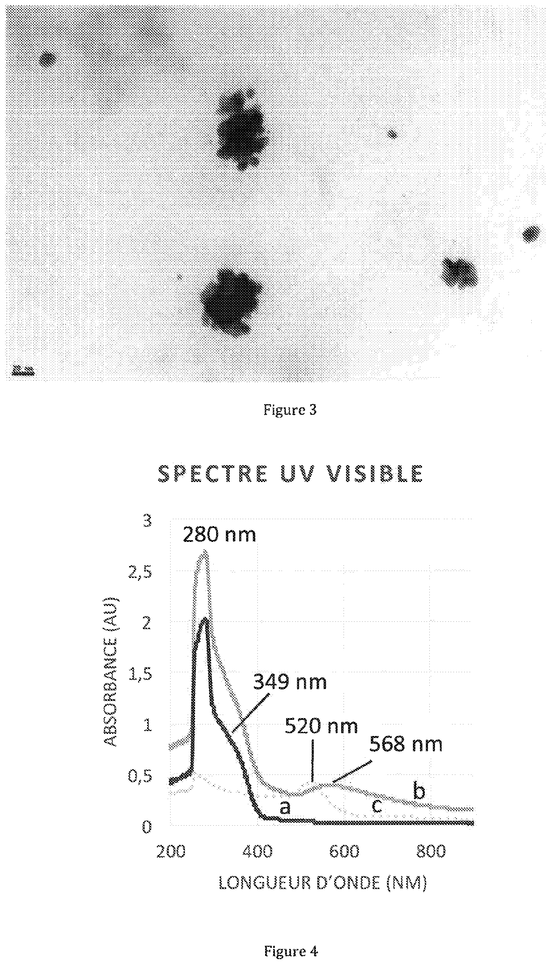

La figure 3 présente des clichés pris en microscopie électronique à transmission de nanoparticules d'or obtenues selon le procédé de l'invention à partir de sels d'or réduits par un extrait total brut de fleurs de *Hypericum lanceolatum.* Les nanoparticules obtenues ont un diamètre de 40 nm et sont anisotropiques en forme de fleurs. Elles sont monodispersées.

La figure 4 présente le spectre UV pour les nanoparticules d'or obtenues par le procédé selon l'invention.

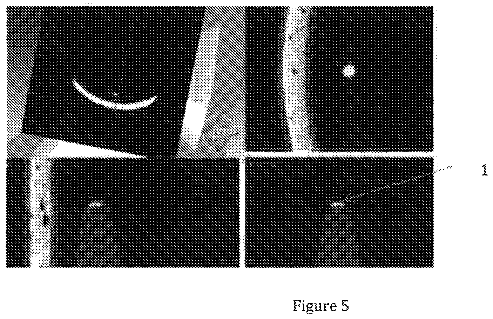

La figure 5 présente le scan des nanoparticules d'or synthétisées par le procédé selon l'invention.

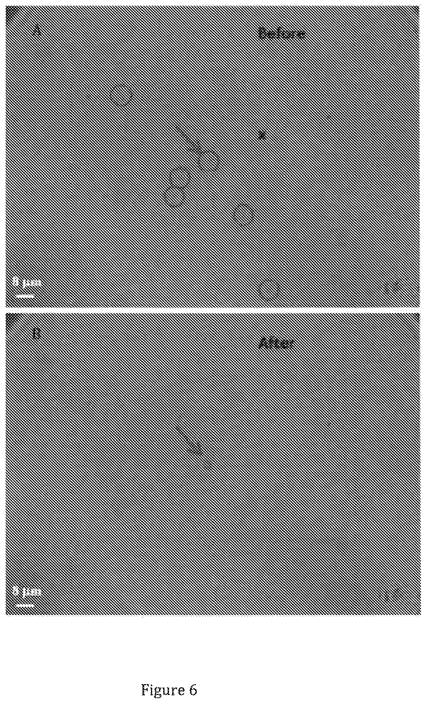

La figure 6 présente en (A) une image optique (champ lumineux) d'une lame de verre avec une nanoparticule colloïdale déposée (certains agrégats absorbants sont marqués de cercles). L'agrégat indiqué par une flèche en (A) et (B) a été ciblé avec la lumière laser (9,5 mW / $\mu$m2 pour 5 s). En (B), une microbulle est formée au niveau de nanoparticules. Par la suite, le laser a été focalisé à la position repérée par X (pas de colloïdes) ; aucune bulle n'est formée dans ce cas.

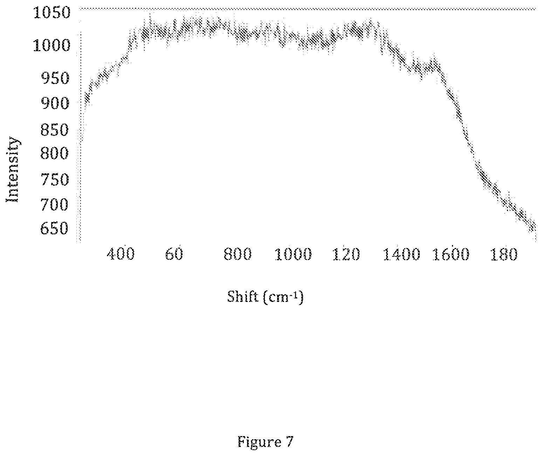

La figure 7 présente les spectres Raman obtenus lors de la focalisation sur un petit agrégat de nanoparticules déposés sur verre et entourés d'eau ($\lambda$exc = 660nm, P = 2mW / $\mu$m2, temps d'intégration de 5 s). Sont représentés, en ordonnées (y) l'intensité en coups/min, et en abscisse (x) le nombre d'onde (cm-1).

Les figures 8 à 12 présentent des clichés pris en MET de coupes de tissus de souris ayant reçu des nanoparticules d'or. Les nanoparticules apparaissent sous forme de points noirs.

Figure 8 : (A) foie non traité par les nanoparticules ; B) Foie traité par les nanoparticules (17mg; 24h).

Figure 9 : (A) Foie traité par les nanoparticules (27mg; 24h ; (B) Foie traité par les nanoparticules (37mg; 24h).

Figure 10 : (A) Poumon non traité par les nanoparticules ; (B) Poumon traité par les nanoparticules (7mg; 24h).

Figure 11 : (A) Rein traité par les nanoparticules (7mg; 24h) ; (B) Rein traité par les nanoparticules (27mg; 24h).

Figure 12 : Rein traité par les nanoparticules (37mg; 24h).

## EXEMPLES

**Exemple** 1 : **Préparation des extraits de plantes de** *Hubertia* **ambavilla** **et de** *Hypericum lanceolatum*

Deux espèces de plantes sont utilisées dans la présente invention. La première est *Hubertia ambavilla* qui est un arbuste endémique de l'île de la Réunion. La deuxième est *Hypericum lanceolatum* qui est une espèce de millepertuis arborescent originaire de l'île de la Réunion. Ces deux plantes sont particulièrement riches en flavonoïdes dont la rutine et la quercétine pour *Hypericum lanceolatum* et l'isoquercétine et l'hyperoside pour *Hubertia ambavilla.*

a) Préparation d'un extrait total brut de plantes

Des plantes fraîchement récoltées sont lavées à l'eau déionisée. 3 grammes sont mélangés à 50 mL d'eau déionisée puis le mélange est chauffé à 60°C pendant 5 min, ce qui permet de libérer la matière biologique par lyse des cellules végétales. Le surnageant est ensuite refroidi à température ambiante puis sur la glace pendant 10 minutes. Le surnageant refroidi est ensuite filtré sur un filtre de porosité de grade 2.

Lorsque le matériel de départ est *Hubertia ambavilla,* l'extrait obtenu est vert.

Lorsque le matériel de départ est *Hypericum lanceolatum,* l'extrait obtenu est marron.

Aucun solvant organique n'est utilisé dans cette préparation.

b) Isolement du totum de flavonoïdes de plantes

La méthode d'extraction utilisée est une méthode de macération à froid. Les plantes sont écrasées sur un tamis à pores de diamètre de 10 mm puis laissées à macérer sous agitation à 150 rpm pendant 20h à température ambiante. Un mélange à quantité égale d'eau et d'éthanol est ajouté au mélange dans un ratio solide/solvant de 1:20 pour obtenir le meilleur rendement possible en composés phénoliques (Cujic N *et al*). Après extraction, les macéras sont filtrés, séchés à basse pression (température maximale du bain : 45°C, pression entre 50 et 150 bars) puis lyophilisés pendant 48h.

Le rendement d'extraction pour *Hubertia ambavilla* dans ces conditions est proche de 50%.

**Exemple 2 : Préparation des nanoparticules d'or par mélange avec des extraits de plantes *Hubertia ambavilla* et de *Hypericum lanceolatum***

a) Préparation des nanoparticules d'or en forme de fleur avec les extraits totaux bruts

50 mL d'une solution aqueuse d'acide chloraurique (HAuCl$_4$) à 1 mM sont refluées sous agitation vigoureuse dans un ballon bicol surmonté d'un réfrigérant à reflux à l'abri de la lumière. Lorsque de fines gouttelettes apparaissent sur les parois, 20 mL d'une solution aqueuse d'extraits totaux brut de plantes sont ajoutés très rapidement. La solution devient alors rapidement bleu nuit en 1 minute. Le ballon est ensuite retiré du bain d'huile et la solution est maintenue sous agitation vigoureuse pendant 15 minutes supplémentaires. La solution est finalement maintenue à 4°C à l'abri de la lumière.

Le diamètre des nanoparticules ainsi obtenues est mesuré comme décrit au paragraphe c) qui suit.

Les nanoparticules obtenues ont un diamètre mesuré en MET d'environ 40 nm.

b) Préparation des nanoparticules d'or sphériques avec un totum de flavonoïdes

4 mL d'une solution aqueuse de totum de flavonoïdes sont refluées sous agitation vigoureuse dans un ballon bicol surmonté d'un réfrigérant à reflux à l'abri de la lumière. Lorsque de fines gouttelettes apparaissent sur les parois, 4 mL d'une solution aqueuse de HAuCl$_4$ sont ajoutés très rapidement. La solution devient alors rapidement rouge brun en 1 minute. Le ballon est ensuite retiré du bain d'huile et la solution est maintenue sous agitation vigoureuse pendant 15 minutes supplémentaires. La solution est finalement maintenue à 4°C à l'abri de la lumière.

Le diamètre des nanoparticules ainsi obtenues est mesuré comme décrit au paragraphe c) qui suit.

Les nanoparticules obtenues ont un diamètre mesurée en MET d'environ 15 nm.

Un ratio molaire spécifique entre les réactifs permet d'obtenir des nanoparticules d'or sphériques. Ce ratio est le suivant :

$$n(\text{flavonoïdes})/n(\text{HAuCl}_4) = 21$$

c) Caractérisation des nanoparticules obtenues en fonction du procédé de préparation

• Diamètre des nanoparticules

Le diamètre des nanoparticules obtenues selon les procédés décrits aux paragraphes a) et b) qui précèdent, est mesuré par microscopie électronique à transmission (MET), par diffraction de la lumière (DLS) et par microscopie à force atomique (AFM).

Pour l'analyse en MET, les images de microscopie électronique ont été enregistrées sur un microscope JEOL JEM 1011 fonctionnant à une tension d'accélération de 100 kV. - carbone et séchés à température ambiante. Pour l'analyse en DLS, la taille et la distribution des particules ont été enregistrées sur un analyseur de granulométrie DLS (Analyseur de taille de particules 90 Plus, Brookhaven Instruments Corporation). Pour l'analyse en AFM, les échantillons ont été caractérisés par un PicoScan II de Molecular Imaging avec une extension MAC pour "mode de prise".

Les images obtenues en MET montrent deux types de particules : des particules sphériques et individuelles de 15 nm de diamètre obtenues avec les extraits de totum flavonoïdes et des nanoparticules non sphériques, en forme de fleur obtenues avec les extraits bruts totaux des plantes. Ces dernières particules en forme de fleurs, obtenues avec l'extrait total brut de *Hubertia ambavilla* ou *Hypericum lanceolatum,* ont un diamètre compris entre 40-80 nm. On note qu'elles sont constituées de particules de taille de 15nm de diamètre, bien individualisées. Les plus petites sont synthétisées avec le totum des flavonoïdes.

Les différences observées entre les deux synthèses laissent penser que l'extrait brut est composé de polyphénols qui polymérisent de façon multidirectionnelle dans l'espace en solution au cours de l'étape de chélation conduisant à la structure de fleur.

Les données obtenues en DLS montrent deux grands groupes de nanoparticules. Le premier groupe est constitué de particules individuelles qui peuvent polymériser pour former le second groupe, des dimères en solution. Les résultats obtenus dans les DLS montrent une taille moyenne de particules nanométriques supérieure à celles obtenues avec les méthodes MET et AFM. Ces différences sont habituellement rapportées dans la littérature (Elia, 2014). Ceci est dû à la technique DLS qui mesure le volume hydrodynamique en considérant le cortège de biomolécules (polyphénols) entourant les particules.

Le traitement de l'image obtenu en AFM donne la taille moyenne des nanoparticules en forme de fleur autour de 33 nm. Les nanoparticules individuelles ont une taille moyenne d'environ 15 nm.

Les tailles des différentes nanoparticules mesurées par les différentes méthodes sont résumées dans le Tableau 1.

AuNP@EBHA = Nanoparticules d'or (AuNP) obtenues en utilisant un extrait brut de *Hubertia ambavilla.*

AuNP@EBHL = AuNP obtenues en utilisant un extrait brut de *Hypericum lanceolatum.*

AuNP@F2HA = AuNP obtenues en utilisant un totum de flavonoides d*'Hubertia ambavilla*

Tableau 1 : Taille des nanoparticules selon la méthode de mesure utilisée.

|  | **AuNP@EBHA** | **AuNP@EBHL** | **AuNP@F2HA** |
|---|---|---|---|
| **AFM** | Large band: 550-590 nm $\lambda max_{SPR}$= 550 nm | Large band: 500-900 nm $\lambda max_{SPR}$= 670 nm | Narrow band $\lambda max_{SPR}$= 530 nm |
| **TEM** | Flower shaped Size: 30-80nm | Flower shaped Size: 30-80nm | Spherical Size:10-15 nm |
| **DLS** | Average size: 109.7 nm Polydispersity 0.19 | Average size: 82.5 nm Polydispersity 0.28 | ND |

• Formes des nanoparticules

Les figures 1 à 3 montrent que des nanoparticules d'or ont bien été obtenues par le procédé selon l'invention. De plus, lorsqu'un extrait total brut de plante est utilisé, les nanoparticules sont anisotropiques et en forme de fleur alors que lorsqu'un totum de flavonoïdes est utilisé, les nanoparticules d'or sont sphériques et plus petites. Les nanoparticules obtenues selon ce procédé sont monodispersées.

Les spectres UV d'un extrait de *Hypericum lanceolatum,* de nanoparticules obtenues par réaction des sels d'or avec un extrait de *Hypericum lanceolatum* et de nanoparticules obtenues par le procédé classique de Turkevich ont été comparés. La figure 4 présente les spectres obtenus, en **a** avec les extraits de *Hypericum lanceolatum,* en **b** les nanoparticules préparées par réaction avec un extrait de *Hypericum lanceolatum* et en **c** les nanoparticules préparées par réaction selon le procédé de Turkevich. Sur le spectre **b,** une bande apparaît à 568 nm confirmant la présente de nanoparticules d'or anisotropiques en forme de fleurs.

• Contraste des nanoparticules étudiées par scanner

La faisabilité de la détection par scanner (CT-scan) des nanoparticules obtenues par le procédé selon l'invention a été vérifiée par tomographie. A cet effet, trois échantillons ont été scannés par tomographie assistée par ordinateur :

- Echantillon n°1 : une goutte de nanoparticules d'or préparées à partir de citrates selon Turkevich.
- Echantillon n°2 : le surnageant (extrait de plante seul),
- Echantillon n°3 : une goutte de nanoparticules d'or préparé à partir d'un extrait brut de *Hypericum lanceolatum,*

Le scanner montre une goutte de nanoparticules synthétisées à partir de sels d'or et d'un extrait de *Hypericum lanceolatum* (échantillon n°3). Après ultracentrifugation, les échantillons n°2 et n°1 ne présente pas de différences de contraste significatives. Par contre, l'échantillon n°3 présente un dépôt noir au fond du tube d'ultracentrifugation présentant un fort contraste (Figure 5). Le contraste radiologique à 80kVp de cet échantillon n°3 est de 621 UH, contre 25 seulement pour le surnageant (échantillon n°2) pour une concentration de 6mg Au/Kg, soit 15 fois inférieure à celle de Boote et *al* (données non montrées).

Boote et *al* ont démontré qu'il existait une relation linéaire entre la concentration de nanoparticules d'or et le contraste radiologique (exprimé en HU). Dans les tests qu'ils ont réalisé *in vitro,* les nanoparticules d'or de 20 nm présentent un contraste radiologique d'environ 10HU à 80 kVp pour une concentration de 90mg Au/Kg. Les expériences menées sur des souris révèlent une accumulation majoritaire des nanoparticules d'or dans le foie, avec une variation du contraste de +22.3 HU (par mg Au absorbé/ lcm3) à 80 kVp and +26.7 HU (par mg Au absorbé/ lcm3) à 140 kVp dans les tissus du foie. Les données de la rate démontrent une variation de valeurs HU de +9.7 HU (mg Au absorbé/ lcm3) à 80 kVp et de +10.1 HU (mg Au absorbé/ lcm3) à 140 kVp. Une autre équipe (Chanda et al) a montré un contraste radiologique d'environ 45HU à 80kVp pour une concentration de 0.016 M [Au] (soient environ 1.54 g Au/Kg)

La comparaison des résultats obtenus par d'autre équipes (présentés ci-dessus) avec ceux obtenus avec les nanoparticules de l'invention montrent que le contraste radiologique obtenu avec les nanoparticules obtenues avec le procédé objet de l'invention est nettement supérieur à ce qui a été décrit antérieurement. Les nanoparticules de l'invention sont donc particulièrement adaptées pour l'imagerie.

• Rendement de synthèse

Les rendements de synthèse des nanoparticules d'or obtenues par le procédé de l'invention utilisant un extrait total brut de plante ou du totum de flavonoïdes de la même plante sont présentés dans le tableau 2 ci-dessous :

Tableau 2. Rendement de synthèse de nanoparticules d'or avec les différents extraits de plantes.

|  | *Hubertia ambavilla* | *Hypericum lanceolatum* |
|---|---|---|
| Extrait total brut | 5 g/L | 2,5 g/L |
| Totum de flavonoïdes | 25 g/L | 30 g/L |

La synthèse avec l'extrait total brut de *Hubertia ambavilla* présente un meilleur rendement que la synthèse avec l'extrait total brut de *Hypericum lanceolatum.*

Le rendement de synthèse de nanoparticules d'or est légèrement supérieur avec le totum de flavonoïdes de *Hypericum lanceolatum* qu'avec celui de *Hubertia ambavilla.*

Les rendements de synthèse de nanoparticules d'or sont plus importants avec les totum de flavonoïdes qu'avec les extraits totaux bruts.

Les rendements obtenus avec les totum de flavonoïdes ont été comparés avec les rendements obtenus avec un seul flavonoïde. Le totum de flavonoïdes de *Hubertia ambavilla* contient majoritairement de l'hyperoside et un peu d'isoquercétine. L'utilisation du totum de flavonoïdes de *Hubertia ambavilla* dans le procédé selon l'invention entraîne l'obtention de nanoparticules d'or sphériques et stables. A l'inverse, la réaction de sels d'or avec de l'isoquercétine seule ou avec de l'hypéroside seul, entraine la synthèse de nanoparticules d'or non stables et un rendement de 20g/L, inférieur au rendement obtenu avec le totum de flavonoïdes. Il y a donc une action synergique entre l'hyperoside et l'isoquercétine.

Le totum de flavonoïdes de *Hypericum lanceolatum* contient majoritairement de la rutine et un peu de quercétine. L'utilisation de ce dernier dans le procédé selon l'invention entraîne l'obtention de nanoparticules d'or sphériques et stables. A l'inverse, la réaction de sels d'or avec de la rutine seule ou avec de la quercétine seule, ne permet pas la synthèse de nanoparticules d'or. Il y a donc une action synergique entre la rutine et la quercétine.

**Exemple 3** : **Etude de la photothermie des nanoparticules d'or *in vitro***

La capacité des nanoparticules à émettre de la chaleur sous irradiation infrarouge a été étudiée *in vitro.* Les AuNP utilisées dans cette expérience ont été obtenues par mélange avec un totum de flavonoïdes de *Hubertia ambavilla.*

Il est connu que les nanoparticules d'or présentent un fort potentiel pour le traitement de tumeurs par hyperthermie. Toutefois, leur efficacité va dépendre de leur taille, de leur forme ou de leur état de surface.

Dans le but d'évaluer la puissance de l'irradiation laser de l'échantillon autour de la résonance plasmonique des nanoparticules employées, et le temps d'exposition requis pour atteindre une température suffisante pour le traitement photothermique des cellules, la génération de microbulles sous l'éclairage continu des vagues a été étudié. Selon Baffou et al. (J. Phys. Chem. C 2014, 118, 4890), la température locale requise pour déclencher la génération de bulles est d'environ 220 ° C.

Résultats

De petits agrégats de nanoparticules ont été visualisés par microscopie optique en utilisant un objectif de 80X, 0,75 NA. Les plus petites taches noires observées en champ clair sont susceptibles de correspondre à des agrégats d'une taille d'environ 200 nm (figure 6A). Des nanoparticules individuelles n'ont pu être localisées en raison de la limite de diffraction. Après avoir enregistré une première image optique avec un laser éteint, la lumière laser (exc = 660 nm) a été focalisée sur l'agrégat pendant un temps fixe. Une deuxième image optique a ensuite été enregistrée après coupure de la lumière laser. Comme le montre la Fig. 6B, une microbulle a été formée avec une exposition de 5 s et une irradiation de 9,5 mW / $\mu$m2. La même expérience a été répétée 5 fois sur d'autres agrégats de taille similaire, entraînant toujours la formation de microbulles pour des durées d'exposition entre 2 et 5 secondes avec la même irradiation laser. Une irradiation plus élevée conduit à des bulles plus grandes.

Des tests négatifs ont également été réalisés: lors de la focalisation de la lumière laser loin de l'agrégat de nanoparticules, aucune bulle n'est formée (position X sur la figure 6A) . Les bulles sont générées au niveau des NP et sont dues à l'absorption plasmonique des NP. De plus, l'irradiation d'agrégats similaires avec 5 mW / $\mu$m2 pendant 30 secondes ne produit pas de microbulles. Le seuil de formation des bulles (T - 220 ° C) est compris entre 5 et 9,5 mW / $\mu$m2 d'irradiation laser. Puisque le seuil se produit à haute température, on pourrait supposer que la température et par conséquent la puissance nécessaire pour induire la mort cellulaire devrait être plus faible. Pour les mesures photothermiques présentées précédemment sur les cellules immobilisées, une irradiation laser> 6 mW / $\mu$m2 a été choisie. Un faible signal Raman (bandes larges de carbone amorphe à - 1300 et - 1550 cm-1) peut être détecté à partir des agrégats NP en utilisant 2 mW / $\mu$m2 pendant 5 sec (Figure 7). Le signal est toujours présent après la formation de la bulle (c'est-à-dire après chauffage), mais avec un fond beaucoup plus bas.

La bande de plasmon des nanoparticules d'or anisotropiques de l'invention se situe entre 600 et 700 nm, décalée de plus de 100 nm par rapport aux nanoparticules individuelles en solution. Ce décalage peut être le résultat de deux phénomènes. Le premier correspond au changement d'environnement lors de l'internalisation des nanoparticules. Dans ce cas, la nanoparticule peut se couvrir de protéines ou d'autres molécules biologiques ce qui va entrainer un décalage vers le rouge de la résonance plasmon. Le deuxième phénomène correspond à l'agrégation des nanoparticules dans le milieu cellulaire confirmant les observations par microscopie en champ sombre. Les résonances plasmon des nanoparticules se rapprochent des plus grandes longueurs d'onde d'irradiation (808nm) réputées moins énergétiques, donc moins destructrices pour l'organisme vivant.

**Exemple 4** : **Etude de la biodistribution** *in vivo* **des nanoparticules d'or**

**Matériel et méthodes**

Modèles animaux : Les souris utilisées dans ce protocole expérimental sont des souris Swiss mâles de 20 à 30g âgées de 6 semaines venant de R. Janvier.

Synthèse de nanoparticules d'or : Les nanoparticules ont été synthétisées par voie écologique conformément aux procédés de la présente invention, et caractérisées par MET, DLS et AFM. Les nanoparticules d'or (AuNP) ont été injectées par voie intraveineuse aux souris pour la réalisation de l'étude de biodistribution. Toutes les AuNP ont été administrées après centrifugation ; elles sont stabilisées en solution aqueuse.

Description du protocole expérimental: Une injection de 200 $\mu$L des différentes solutions a été réalisée par voie intraveineuse. Pour une souris d'environ 24g, 200$\mu$L ont été administrés.

Les solutions suivantes ont été injectées selon les protocoles déjà décrits par Boote et al, 2010 et Chanda et al, 2014 :

- NaCl (6 souris)
- Produit 1 (AuNP@EBHL = AuNP obtenues en utilisant un extrait brut de *Hypericum lanceolatum*)

> Groupe A (6 souris) = une injection unique **7mg/ml**
> Groupe B (6 souris) = une injection unique **2mg/ml**

- Produit 2 (AuNP@EBHA_A = AuNP obtenues en utilisant un extrait brut de *Hubertia ambavilla*)

> Groupe A (6 souris) = une injection unique **7mg/ml**
> Groupe B (6 souris) = une injection unique **2mg/ml**

> Produit 3 (AuNP@F2HA = AuNP obtenues en utilisant un totum de flavonoides *d'Hubertia ambavilla*)

Groupe A (6 souris) = une injection unique **7mg/ml**
Groupe B (6 souris) = une injection unique **2mg/ml**

Soit un total de 42 souris.

Ces souris ont ensuite été séparées en 2 groupes pour chaque concentration de chaque produit. Le premier groupe a été sacrifié au bout de 6h (3 souris par produit et par concentration soit un total de 27 souris) et le deuxième groupe a été sacrifié au bout de 24h.

Enfin les différents organes a été prélevés (Foie, Cerveau, Rein, Rate, Poumon et Coeur) afin d'étudier la biodistribution des différents produits.

**Résultats**

Après injection des AuNP par voie intraveineuse, les extraits d'organes ont été analysés par MET afin de localiser les nanoparticules.

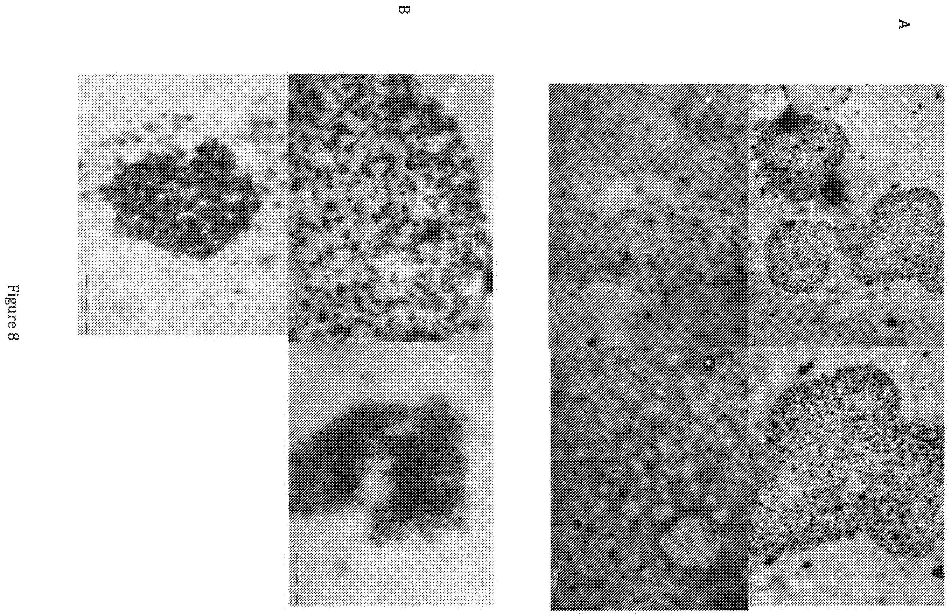

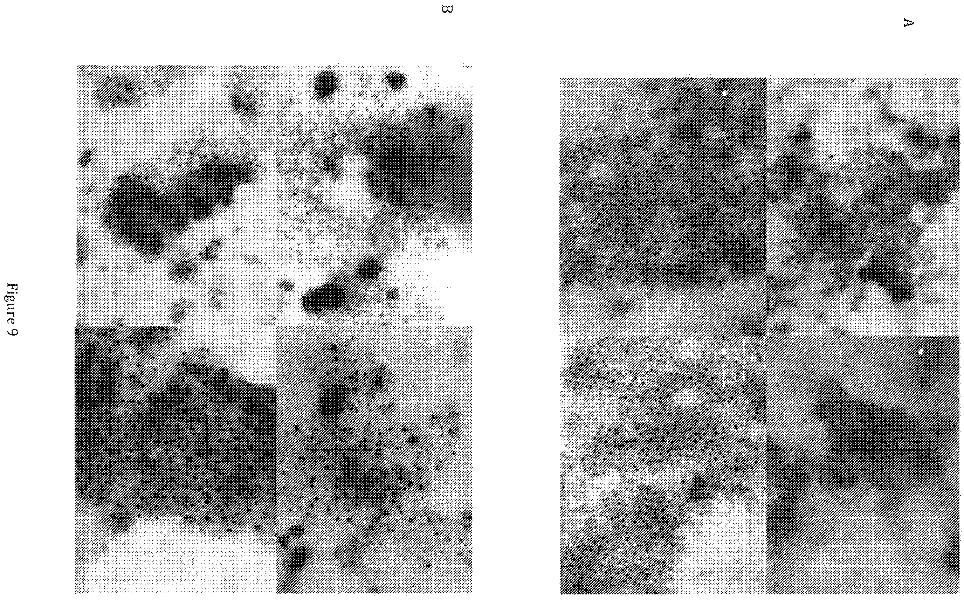

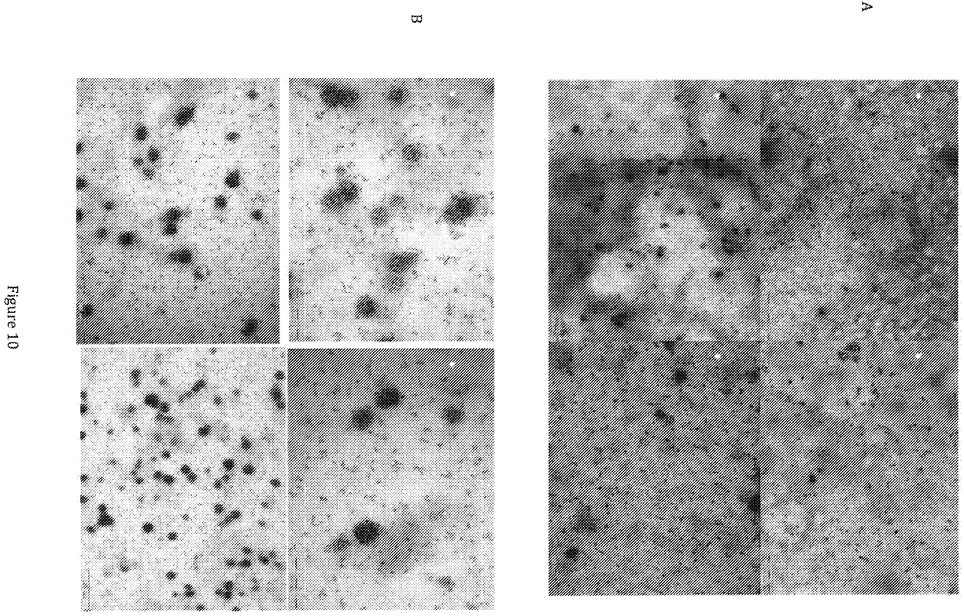

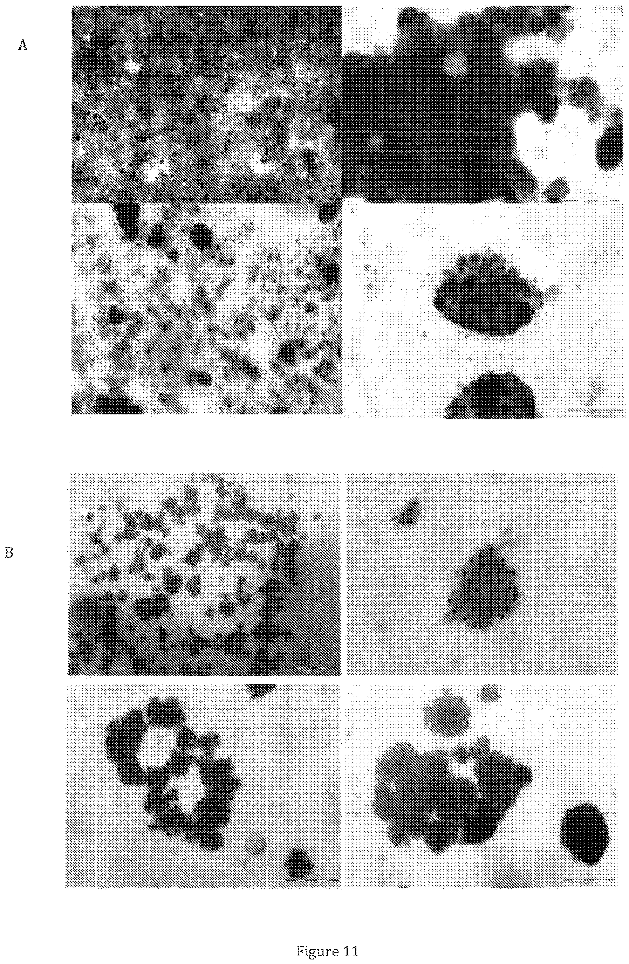

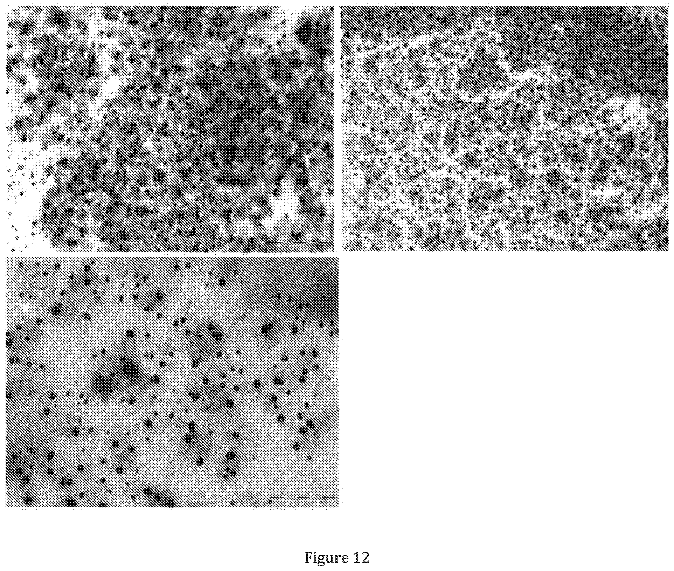

Les nanoparticules ont été retrouvées accumulées dans les organes suivants : poumons, reins et foie. Les résultats correspondant aux souris injectées avec le produit 3 sont illustrés aux Figures 8 à 12. Une biodistribution similaire est obtenue avec les produits 1 et 2 (données non présentées).

Ces résultats montrent que la quantité de AuNP retrouvées dans les différents organes est proportionnelle à la quantité administrée.

**Références** :

N. Ćujić, K. Šavikin, T. Janković, D. Pljevljakusic, G. Zdunić, S. Ibric, 2016. Optimization of polyphenols extraction from dried chokeberry using maceration as traditional technique, Food Chemistry, 194, 135-142

T. Stylianopoulos, and R. K. Jain "Design considerations for nanotherapeutics in oncology." Nanomedicine: Nanotechnology, Biology and Medicine 11(8): 1893-1907.

J. Turkevich, P. C. Stevenson and J. Hiller, Discuss. "A study of the nucleation and growth processes in the synthesis of colloidal gold", Faraday Soc. 1951, 11, 55.

Jabeen F, Najam-ul-Haq M, Javeed R, Huck CW, Bonn GK. Au-nanomaterials as a superior choice for near-infrared photothermal therapy. Molecules (Basel, Switzerland). 2014;19:20580-93.

Jiang Y, Fei W, Cen X, Tang Y, Liang X. Near-infrared Light Activatable Multimodal Gold Nanostructures Platform: An Emerging Paradigm for Cancer Therapy. Current cancer drug targets. 2015;15:406-22.

Shanmugam V, Selvakumar S, Yeh CS. Near-infrared light-responsive nanomaterials in cancer therapeutics. Chemical Society reviews. 2014;43:6254-87.

Arifin DR, Long CM, Gilad AA, Alric C, Roux S, Tillement O, Link TW, Arepally A, Bulte JW. Trimodal gadolinium-gold microcapsules containing pancreatic islet cells restore normoglycemia in diabetic mice and can be tracked by using US, CT, and positive-contrast MR imaging. Radiology. 2011;260:790-8.

Song J, Qu J, Swihart MT, Prasad PN. Near-IR responsive nanostructures for nanobiophotonics: emerging impacts on nanomedicine. Nanomedicine : nanotechnology, biology, and medicine. 2016;12:771-88.

Ke H, Yue X, Wang J, Xing S, Zhang Q, Dai Z, Tian J, Wang S, Jin Y. Gold nanoshelled liquid perfluorocarbon nanocapsules for combined dual modal ultrasound/CT imaging and photothermal therapy of cancer. Small. 2014;10:1220-7.

Huang, X.; El-Sayed, M.A. Plasmonic photo-thermal therapy (PPTT).Alex. J. Med.2011,47, 1-9

Mackey, M.A.; Ali, M.R.K.; Austin, L.A.; Near, R.D.; El-Sayed, M.A. The most effective gold nanorod size for plasmonic photothermal therapy: Theory and in vitro experiments.J. Phys. Chem. B2014,118, 1319-1326.

E. S. Shibu, M. Hamada, N. Murase, V. Biju, J. Photoch. Photobio. C 2013,15, 53.

Nabil, M.; Decuzzi, P.; Zunino, P. Modelling mass and heat transfer in nano-based cancer hyperthermia.R. Soc. Open Sci.2015,2, 150447

Peeters, S.; Kitz, M.; Preisser, S.; Wetterwald, A.; Rothen-Rutishauser, B.; Thalmann, G.N.; Brandenberger, C.; Bailey, A.; Frenz, M. Mechanisms of nanoparticle-mediated photomechanical cell damage. Biomed. Opt. Express 2012,3, 435-446

Li, D.; Wang, G.X.; He, Y.L.; Wu, W.J.; Chen, B. A three-temperature model of selective photothermolysis forlaser treatment of port wine stain containing large malformed blood vessels. Appl. Therm. Eng.2014,65,308-321.

Clark, C.D.; Denton, M.L.; Thomas, R.J. Mathematical model that describes the transition from thermal to photochemical damage in retinal pigment epithelial cell culture.J. Biomed. Opt.2011,16, 020504.

Xu L, Wan C, Du J, Li H, Liu X, Yang H, Li F., Synthesis, characterization, and in vitro evaluation of targeted gold nanoshelled poly(D,L-lactide-co-glycolide) nanoparticles carrying anti p53 antibody as a theranostic agent for ultrasound contrast imaging and photothermal therapy. J Biomater Sci Polym Ed. 2017 Jan 3:1-37.

Krishnaswamy, K., Vali, H., & Orsat, V. (2014). Value-adding to grape waste: Green synthesis of gold nanoparticles. Journal of Food Engineering, 142, 210-220.

Elia, P., Zach, R., Hazan, S., Kolusheva, S., Porat, Z. E., & Zeiri, Y. (2014). Green synthesis of gold nanoparticles using plant extracts as reducing agents. International journal of nanomedicine, 9, 4007.

Boote, E., Fent, G., Kattumuri, V., Casteel, S., Katti, K., Chanda, N., ... & Churchill, R. (2010). Gold nanoparticle contrast in a phantom and juvenile swine: models for molecular imaging of human organs using x-ray computed tomography. Academic radiology, 17(4), 410-417.

Chanda, N., Upendran, A., Boote, E. J., Zambre, A., Axiak, S., Selting, K., ... & Singh, J. (2014). Gold nanoparticle based X-ray contrast agent for tumor imaging in mice and dog: a potential nanoplatform for computer tomography theranostics. Journal of biomédical nanotechnology, 10(3), 383-392.

Frens, G. (1973). Controlled nucleation for the regulation of the particle size in monodisperse gold suspensions. Nature, 241(105), 20-22.

Turkevich, J., Stevenson, P. C., & Hillier, J. (1951). A study of the nucleation and growth processes in the synthesis of colloidal gold. Discussions of the Faraday Society, 11, 55-75.

Zhao, P., Li, N., & Astruc, D. (2013). State of the art in gold nanoparticle synthesis. Coordination Chemistry Reviews, 257(3), 638-665.

## Revendications

1. Procédé écologique de préparation de nanoparticules d'or biocompatibles et stables comprenant :

   a. La préparation d'au moins un extrait de plante riche en flavonoïdes
   b. Le mélange d'au moins un desdits extraits de plante avec une solution aqueuse d'au moins un sel d'or
   **caractérisé en ce que** l'extrait de plante riche en flavonoïdes est un extrait de *Hubertia ambavilla* ou de *Hypericum lanceolatum.*

2. Procédé selon la revendication 1 **caractérisé en ce que** l'extrait de plante riche en flavonoïdes est un extrait total brut de ladite plante ou un totum de flavonoïdes de ladite plante.

3. Procédé selon la revendication 1 ou 2 **caractérisé en ce que** l'extrait de plante comprend des flavonoïdes choisis parmi la rutine, la quercétine, l'hyperoside et l'isoquercétine ou une combinaison d'au moins deux parmi ceux-ci.

**4.** Procédé selon l'une quelconque des revendications précédentes **caractérisé en ce que** les nanoparticules ont un diamètre compris entre 5 et 100 nm mesuré par microscopie électronique à transmission.

**5.** Procédé selon l'une quelconque des revendications précédentes **caractérisé en ce que** les nanoparticules sont sphériques, lesdites nanoparticules sphériques étant obtenues par mélange d'un totum de flavonoïdes de plante avec une solution aqueuse d'au moins un sel d'or.

**6.** Procédé selon l'une quelconque des revendications 1 à 5 **caractérisé en ce que** les nanoparticules sont anisotropiques en forme de fleur, lesdites nanoparticules d'or en forme de fleur étant obtenues par mélange d'un extrait total brut de plante avec une solution aqueuse d'au moins un sel d'or.

**7.** Nanoparticule d'or anisotropique en forme de fleur comprenant un mélange d'or et d'extrait brut de *Hubertia ambavilla* ou de *Hypericum lanceolatum.*

**8.** Nanoparticule selon la revendication 7 dont le diamètre est compris entre 40 à 80 nm lorsqu'il est mesuré par microscopie électronique à transmission.

**9.** Nanoparticule selon l'une des revendications 7 ou 8, ladite nanoparticule étant fonctionalisée par des protéines, en particulier des anticorps, des polymères ou des sucres.

**10.** Nanoparticule selon l'une des revendications 7 à 9, ou susceptible d'être obtenue par le procédé défini à l'une des revendications 1 à 6, pour son utilisation comme médicament.

**11.** Nanoparticules selon la revendication 10 pour son utilisation selon la revendication 10 pour traiter le cancer.

**12.** Nanoparticules selon la revendication 11 pour son utilisation selon la revendication 11 dans laquelle le cancer à traiter est choisi parmi le cancer du foie, la cancer du poumon et le cancer du rein.

**13.** Utilisation d'une nanoparticule selon l'une des revendications 7 à 9, ou susceptible d'être obtenue par le procédé défini à l'une des revendications 1 à 6, comme outil de diagnostique ou d'imagerie.

**14.** Composition diagnostique et/ou thérapeutique comprenant des nanoparticules selon l'une des revendications 7 à 9, ou susceptible d'être obtenue par le procédé défini à l'une des revendications 1 à 6.

**15.** Composition cosmétique comprenant des nanoparticules selon l'une des revendications 7 à 9, ou susceptible d'être obtenue par le procédé défini à l'une des revendications 1 à 6.

**Patentansprüche**

**1.** Ökologisches Verfahren zur Herstellung von biokompatiblen, stabilen Goldnanopartikeln, das folgende Schritte umfasst:

A. Die Herstellung von zumindest einem Pflanzenextrakt, das reich an Flavonoiden ist.
B. Die Mischung von zumindest einem dieser Pflanzenextrakte mit einer wässrigen Lösung von zumindest einem Goldsalz, **dadurch gekennzeichnet, dass** das flavonoidreiche Pflanzenextrakt ein Extrakt von Hubertia ambavilla oder von Hypericum lanceolatum ist.

**2.** Verfahren nach Anspruch 1, **dadurch gekennzeichnet, dass** das flavonoidreiche Pflanzenextrakt ein Gesamtrohextrakt dieser Pflanze oder ein Totum von Flavonoiden dieser Pflanze ist.

**3.** Verfahren nach Anspruch 1 oder 2, **dadurch gekennzeichnet, dass** das Pflanzenextrakt Flavonoide umfasst, die aus Rutin, Quercetin, Hyperosid und Isoquercetin oder einer Kombination von zumindest zwei dieser Flavonoide ausgewählt werden.

**4.** Verfahren nach einem der vorhergehenden Ansprüche, **dadurch gekennzeichnet, dass** die Nanopartikel bei einer Messung per Transmissionselektronenmikroskopie einen Durchmesser zwischen 5 und 100 nm aufweisen.

**5.** Verfahren nach einem der vorhergehenden Ansprüche, **dadurch gekennzeichnet, dass** die Nanopartikel kugelförmig sind, wobei diese kugelförmigen Nanopartikel durch Mischen eines Totums an Pflanzenflavonoiden mit einer wässrigen Lösung von zumindest einem Goldsalz erhalten werden.

**6.** Verfahren nach einem der Ansprüche 1 bis 5, **dadurch gekennzeichnet, dass** die Nanopartikel anisotrop und blütenförmig sind, wobei die blütenförmigen Goldnanopartikel durch Mischen eines Gesamtrohextrakts einer Pflanze mit einer wässrigen Lösung von zumindest einem Goldsalz erhalten werden.

**7.** Anisotropes, blumenförmiges Goldnanopartikel, welches eine Mischung aus Gold und dem Rohextrakt von Hubertia ambavilla oder Hypericum lanceolatum umfasst.

**8.** Nanopartikel nach Anspruch 7, das bei einer Messung per Transmissionselektronenmikroskopie einen Durchmesser zwischen 40 und 80 nm aufweist.

**9.** Nanopartikel nach einem der Ansprüche 7 oder 8, wobei das Nanopartikel durch Proteine, insbesondere Antikörper, Polymere oder Zucker, funktionalisiert wird.

**10.** Nanopartikel nach einem der Ansprüche 7 oder 9, oder Nanopartikel, das durch das in einem der Ansprüche 1 bis 6 definierten Verfahren für dessen Verwendung als Medikament erhalten werden kann.

**11.** Nanopartikel nach Anspruch 10, die zur Behandlung von Krebs gemäß Anspruch 10 verwendet werden.

**12.** Nanopartikel nach Anspruch 11 zur Verwendung gemäß Anspruch 11, bei der der zu behandelnde Krebs ausgewählt wird aus Leberkrebs, Lungenkrebs und Nierenkrebs.

**13.** Verwendung eines Nanopartikels nach einem der Ansprüche 7 bis 9, oder eines Nanopartikels, das durch das in einem der Ansprüche 1 bis 6 definierten Verfahren erhalten werden kann, als Diagnose- oder Bildgebungsinstrument.

**14.** Diagnostische und/oder therapeutische Zusammensetzung, welche Nanopartikel nach einem der Ansprüche 7 bis 9 umfasst, oder die über das in einem der Ansprüche 1 bis 6 definierten Verfahren erhalten werden kann.

**15.** Kosmetische Zusammensetzung, die Nanopartikel nach einem der Ansprüche 7 bis 9 umfasst, oder die über das in einem der Ansprüche 1 bis 6 definierten Verfahren erhalten werden kann.

**Claims**

**1.** An ecological method for preparing biocompatible and stable gold nanoparticles comprising:

a. Preparing at least one -plant extract rich in flavonoids
b. Mixing at least one of said plant extracts with an aqueous solution of at least one gold salt **characterized in that** the -plant extract rich in flavonoids is an extract of Hubertia ambavilla or Hypericum lanceolatum.

**2.** The method according to claim 1 **characterized in that** the plant extract rich in flavonoids is a total crude extract of said plant or a totum of flavonoids of said plant.

**3.** The method according to claim 1 or 2, **characterized in that** the plant extract comprises flavonoids selected from rutin, quercetin, hyperoside and isoquercetin or a combination of at least two of these.

**4.** The method according to any one of the preceding claims, **characterized in that** the nanoparticles have a diameter between 5 and 100nm measured by transmission electron microscopy.

**5.** The method according to any one of the preceding claims, **characterized in that** the nanoparticles are spherical, said spherical nanoparticles being obtained by mixing a totum of plant flavonoids with an aqueous solution of at least one gold salt.

**6.** The method according to any one of claims 1 to 5, **characterized in that** the nanoparticles are anisotropic and flower-shaped, said flower-shaped gold nanoparticles being obtained by mixing a total crude plant extract with an aqueous solution of at least one gold salt.

7. An anisotropic flower-shaped gold nanoparticle comprising a mixture of gold and a crude extract of Hubertia ambavilla or Hypericum lanceolatum.

8. The nanoparticle according to claim 7, having a diameter between 40 and 80nm when measured by transmission electron microscopy.

9. The nanoparticle according to one of claims 7 or 8, said nanoparticle being functionalized by proteins, in particular antibodies, polymers or sugars.

10. The nanoparticle according to one of claims 7 to 9, or obtainable with the method defined in one of claims 1 to 6, for use as a medicine.

11. Nanoparticles according to claim 10 for its use according to claim 10, for treating cancer.

12. The nanoparticles according to claim 11 for its use according to claim 11, in which the cancer to be treated is selected from liver cancer, lung cancer and kidney cancer.

13. The use of a nanoparticle according to one of claims 7 to 9, or obtainable with the method defined in one of claims 1 to 6, as a diagnostic or imaging tool.

14. A diagnostic and/or therapeutic composition comprising nanoparticles according to one of claims 7 to 9, or obtainable with the method defined in one of claims 1 to 6.

15. A cosmetic composition comprising nanoparticles according to one of claims 7 to 9, or obtainable with the method defined in one of claims 1 to 6.

Figure 1

Figure 2

Figure 3

SPECTRE UV VISIBLE

Figure 4

Figure 5

Figure 6

Figure 7

A

B

Figure 8

A

B

Figure 9

A

B

Figure 10

Figure 11

Figure 12

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2005095031 A
- US 8333994 B

**Littérature non-brevet citée dans la description**

- **KUMAR et al.** *J Chem Technol Biotechnol,* 2009
- **BAFFOU et al.** *J. Phys. Chem. C,* 2014, vol. 118, 4890,
- **N. CUJIC ; K. ŠAVIKIN ; T. JANKOVIC ; D. PLJEVLJAKUŠIC ; G. ZDUNIC ; S. IBRIC.** Optimization of polyphenols extraction from dried chokeberry using maceration as traditional technique. *Food Chemistry,* 2016, vol. 194, 135-142
- **T. STYLIANOPOULOS ; R. K. JAIN.** Design considerations for nanotherapeutics in oncology. *Nanomedicine: Nanotechnology, Biology and Medicine,* vol. 11 (8), 1893-1907
- **J. TURKEVICH ; P. C. STEVENSON ; J. HILLER.** A study of the nucleation and growth processes in the synthesis of colloidal gold. *Faraday Soc.,* 1951, vol. 11, 55
- **JABEEN F ; NAJAM-UL-HAQ M ; JAVEED R ; HUCK CW ; BONN GK.** Au-nanomaterials as a superior choice for near-infrared photothermal therapy. *Molecules (Basel, Switzerland),* 2014, vol. 19, 20580-93
- **JIANG Y ; FEI W ; CEN X ; TANG Y ; LIANG X.** Near-infrared Light Activatable Multimodal Gold Nanostructures Platform: An Emerging Paradigm for Cancer Therapy. *Current cancer drug targets,* 2015, vol. 15, 406-22
- **SHANMUGAM V ; SELVAKUMAR S ; YEH CS.** Near-infrared light-responsive nanomaterials in cancer therapeutics. *Chemical Society reviews,* 2014, vol. 43, 6254-87
- **ARIFIN DR ; LONG CM ; GILAD AA ; ALRIC C ; ROUX S ; TILLEMENT O ; LINK TW ; AREPALLY A ; BULTE JW.** Trimodal gadolinium-gold microcapsules containing pancreatic islet cells restore normoglycemia in diabetic mice and can be tracked by using US, CT, and positive-contrast MR imaging. *Radiology,* 2011, vol. 260, 790-8
- **SONG J ; QU J ; SWIHART MT ; PRASAD PN.** Near-IR responsive nanostructures for nanobiophotonics: emerging impacts on nanomedicine. *Nanomedicine : nanotechnology, biology, and medicine,* 2016, vol. 12, 771-88

- **KE H ; YUE X ; WANG J ; XING S ; ZHANG Q ; DAI Z ; TIAN J ; WANG S ; JIN Y.** Gold nanoshelled liquid perfluorocarbon nanocapsules for combined dual modal ultrasound/CT imaging and photothermal therapy of cancer. *Small,* 2014, vol. 10, 1220-7
- **HUANG, X. ; EL-SAYED, M.A. ; ALEX.** Plasmonic photo-thermal therapy (PPTT). *J. Med.,* 2011, vol. 47, 1-9
- **MACKEY, M.A. ; ALI, M.R.K. ; AUSTIN, L.A. ; NEAR, R.D. ; EL-SAYED, M.A.** The most effective gold nanorod size for plasmonic photothermal therapy: Theory and in vitro experiments. *J. Phys. Chem. B,* 2014, vol. 118, 1319-1326
- **E. S. SHIBU ; M. HAMADA ; N. MURASE ; V. BIJU.** *J. Photoch. Photobio. C,* 2013, vol. 15, 53
- **NABIL, M. ; DECUZZI, P. ; ZUNINO, P.** Modelling mass and heat transfer in nano-based cancer hyperthermia. *R. Soc. Open Sci.,* 2015, vol. 2, 150447
- **PEETERS, S. ; KITZ, M. ; PREISSER, S. ; WETTERWALD, A. ; ROTHEN-RUTISHAUSER, B. ; THALMANN, G.N. ; BRANDENBERGER, C. ; BAILEY, A. ; FRENZ, M.** Mechanisms of nanoparticle-mediated photomechanical cell damage. *Biomed. Opt. Express,* 2012, vol. 3, 435-446
- **LI, D. ; WANG, G.X. ; HE, Y.L. ; WU, W.J. ; CHEN, B.** A three-temperature model of selective photothermolysis forlaser treatment of port wine stain containing large malformed blood vessels. *Appl. Therm. Eng.,* 2014, vol. 65, 308-321
- **CLARK, C.D. ; DENTON, M.L. ; THOMAS, R.J.** Mathematical model that describes the transition from thermal to photochemical damage in retinal pigment epithelial cell culture. *J. Biomed. Opt.,* 2011, vol. 16, 020504
- **XU L ; WAN C ; DU J ; LI H ; LIU X ; YANG H ; LI F.** Synthesis, characterization, and in vitro evaluation of targeted gold nanoshelled poly(D,L-lactide-co-glycolide) nanoparticles carrying anti p53 antibody as a theranostic agent for ultrasound contrast imaging and photothermal therapy. *J Biomater Sci Polym Ed.,* 03 Janvier 2017, 1-37

- **KRISHNASWAMY, K. ; VALI, H. ; ORSAT, V.** Value-adding to grape waste: Green synthesis of gold nanoparticles. *Journal of Food Engineering,* 2014, vol. 142, 210-220
- **ELIA, P. ; ZACH, R. ; HAZAN, S. ; KOLUSHEVA, S. ; PORAT, Z. E. ; ZEIRI, Y.** Green synthesis of gold nanoparticles using plant extracts as reducing agents. *International journal of nanomedicine,* 2014, vol. 9, 4007
- **BOOTE, E. ; FENT, G. ; KATTUMURI, V. ; CASTEEL, S. ; KATTI, K. ; CHANDA, N. ; CHURCHILL, R.** Gold nanoparticle contrast in a phantom and juvenile swine: models for molecular imaging of human organs using x-ray computed tomography. *Academic radiology,* 2010, vol. 17 (4), 410-417
- **CHANDA, N. ; UPENDRAN, A. ; BOOTE, E. J. ; ZAMBRE, A. ; AXIAK, S. ; SELTING, K. ; SINGH, J.** Gold nanoparticle based X-ray contrast agent for tumor imaging in mice and dog: a potential nanoplatform for computer tomography theranostics. *Journal of biomédical nanotechnology,* 2014, vol. 10 (3), 383-392
- **FRENS, G.** Controlled nucleation for the regulation of the particle size in monodisperse gold suspensions. *Nature,* 1973, vol. 241 (105), 20-22
- **TURKEVICH, J. ; STEVENSON, P. C. ; HILLIER, J.** A study of the nucleation and growth processes in the synthesis of colloidal gold. *Discussions of the Faraday Society,* 1951, vol. 11, 55-75
- **ZHAO, P. ; LI, N. ; ASTRUC, D.** State of the art in gold nanoparticle synthesis. *Coordination Chemistry Reviews,* 2013, vol. 257 (3), 638-665